# FASCICULE DE BREVET EUROPEEN

(11) **EP 3 060 228 B1**
(45) Date de publication et mention de la délivrance du brevet: **03.04.2019**
(21) Numéro de dépôt: 14790520.2
(22) Date de dépôt: 25.09.2014
(51) Int. Cl.: A61K 36/185, A61K 8/97, A61Q 19/00, A23D 9/007, A61Q 19/06, A61K 9/00, C11B 3/00, A23L 33/10, A61P 17/02

(54) **EXTRAIT LIPIDIQUE DE GRAINES DE PASSIFLORES**
FETTEXTRAKT AUS MARACUJASAMEN
LIPID EXTRACT OF PASSION FRUIT SEEDS

(30) Priorité: 25.09.2013 FR 1359252
(43) Date de publication de la demande: 31.08.2016
(73) Titulaire: Laboratoires Expanscience, 92048 Paris La Défense Cedex (FR)
(72) Inventeur: LECLERE-BIENFAIT, Sophie, F-28100 Dreux (FR); BREDIF, Stéphanie, F-28210 Croisilles (FR); DEBROCK, Sébastien, F-28210 St Martin de Nigelles (FR); GARNIER, Sébastien, F-06650 Le Rouret (FR)
(74) Mandataire: Regimbeau
(86) Numéro de dépôt international: PCT/EP2014/070463
(87) Numéro de publication internationale: WO 2015/044254

(56) Documents cités:
- EP-A1- 1 541 158
- US-A1- 2011 159 074
- MALACRIDA CASSIA ROBERTA ET AL: "Yellow Passion Fruit Seed Oil (Passiflora edulis f. flavicarpa): Physical and Chemical Characteristics", BRAZILIAN ARCHIVES OF BIOLOGY AND TECHNOLOGY, vol. 55, no. 1, janvier 2012 (2012-01), pages 127-134, XP009177810,
- LIU SHUCHENG ET AL: "Physical and chemical analysis of Passiflora seeds and seed oil from China", INTERNATIONAL JOURNAL OF FOOD SCIENCES AND NUTRITION, vol. 59, no. 7-8, 2008, pages 706-715, XP009177808, ISSN: 0963-7486
- Anonymous: "Lipex TM Omega Passiflora", , 1 août 2008 (2008-08-01), XP002723949, Extrait de l'Internet: URL:http://www.aak.com/Global/Products/Bea uty%20and%20personal%20care/Emollients-Ome ga%20Oils/aak-lfc_lipex_omega_Passiflora_0 808.pdf [extrait le 2014-05-02]

## Description

L'invention se rapporte à extrait lipidique de graines de Passiflores, *Passiflora incarnata* et *Passiflora edulis* et préférentiellement *Passiflora edulis* et à une composition cosmétique, dermatologique ou nutraceutique comprenant un excipient approprié et ledit extrait lipidique de graines de Passiflores, *Passiflora incarnata* et *Passiflora edulis.* En effet, les inventeurs ont découvert, de manière surprenante, que les extraits lipidiques de Passiflores et préférentiellement ceux de *Passiflora edulis* présentent des propriétés cosmétiques, dermatologiques ou nutraceutiques intéressantes.

L'invention a également pour objet un procédé d'extraction d'un extrait lipidique de graines de Passiflore, ainsi que l'extrait susceptible d'être obtenu par ledit procédé. L'invention concerne également une telle composition ou un tel extrait pour son utilisation dans la prévention ou le traitement des troubles ou pathologies de la peau, des muqueuses ou des phanères. L'invention concerne enfin un procédé de soin cosmétique de la peau, des phanères ou des muqueuses, en vue d'améliorer leur état ou leur aspect, consistant à administrer une telle composition ou un tel extrait.

### LES PASSIFLORES

La famille des passiflores (*Passiflora*) est constituée d'environ 500 espèces. Les espèces sont souvent distribuées dans les régions chaudes tempérées et tropicales et notamment sur le continent américain, mais sont plutôt rares en Asie, Australie et Afrique tropicale.

### Botanique

Les plants se présentent sont forme d'arbrisseaux ou herbes grimpantes. Les feuilles sont alternes, parfois simples, lobées ou palmées. Les fleurs peuvent attendre 9 cm de diamètre sont bisexuelles ou unisexuelles et régulières. Elles sont blanches et violettes et présentent des appendices pétaloïdes fins, garnis d'appendices filiformes symbolisant la couronne d'épines du Christ. Le fruit de 4 à 5 cm de longueur est ovalaire et souvent de couleur jaune à orangé.

Les espèces les plus répandues sont notamment *P. incarnata et P. edulis.*

### Eléments de Phytochimie

*P. incarnata* : les constituants majeurs sont représentés par la famille des flavonoïdes qui sont présentes en grande quantité dans les feuilles. Elle contient une forte teneur en isovitexine. Elle contient également en faible quantité des alcaloïdes indoliques simples (harmane, harmine...), des sucres comme le raffinose, le saccharose, fructose, glucose, et des huiles essentielles et du maltol décrit comme la molécule qui serait à l'origine des effets sédatifs et anti-convulsifiants attribués à cette plante.

*P. edulis* : à partir d'un extrait méthanolique de feuilles séchées a été identifié un composé spécifique, la passiflorine - cyclopropane triterpène glycoside (E. Bombardelli et al., 1975). Elle contient de l'isoorientine, flavonoïde qui n'est pas retrouvée dans *P. incarnata* ainsi que des traces d'huile essentielle et d'alcaloïdes identiques à *P. incarnata.* La pulpe du fruit contient des flavonoïdes, schaftoside, isoschaftoside, isoorientine, orientine, isovitexine, dérivés de lutéoline (M.L. Zeraik, J.H. Yariwake - 2010), de l'acide ascorbique (environ 60 mg/100 g). La pulpe contient également des dérivés cyanogéniques glycosylés : la prunasine, la sambunigrine et l'amygdaline, ainsi que récemment identifiés, deux mandelonitrile β-rutinosides (D. Chassagne and J. Crouzet, 1998 ; D.S. Seigler, 2002).

### Toxicologie

Des constituants cyanogéniques sont présents essentiellement dans les parties aériennes de différentes variétés de passiflore.

### Caractéristiques de la graine

Les graines constituent 6 à 12% du fruit de *P. Edulis* et elles contiennent :
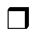 *des polyphénols* dont le Piceatannol (structure proche du resveratrol) et son dimère la scirpusine B (S. Sano ; K. Sugiyama ; T. Ito, 2011), substances à effets vasorelaxant et anti-oxydant.
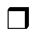 *de l'huile,* 18% par solvant contenant des phytostérols (0.2% dont campestérol, stigmastérol, sitostérol, avenasterol) ; 60 à 73% d'acide linoléique (oméga 6), 14% à 20% d'acide oléique et 465 ppm de tocophérols (G. Piombo, N. Barouh et al, 2006 ; R. de V.V. Lopes et al.).
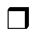 *Des sucres et des protéines*

### ART ANTERIEUR

### Usage Alimentaire

Le fruit semble être consommé depuis la préhistoire. Au XVIème siècle au Pérou, les magnifiques fleurs de passiflores étaient déjà considérées comme un remède et de nombreuses espèces de passiflores sont toujours utilisées dans de nombreux pays dans les pratiques thérapeutiques courantes.

### Usage Médical

Les passiflores (souvent les parties aériennes et parfois les fruits) sont souvent utilisées partout dans le monde comme anxiolytique, sédatif, diurétique et analgésique (toutes les descriptions dans « Passiflora : review update. K. Dhawan, S. Dhawan, A. Sharma, 2004 »). Le maltol et certains de ses dérivés seraient à l'origine de cet effet sédatif.

Cette activité serait plus constante et plus significative pour *P. incarnata.* Des extraits de *P. incarnata* seraient capables de réverser la dépendance à la morphine. Des effets anti-inflammatoires ont également été mis en évidence pour des extraits de feuilles de *P. edulis.*

Les différentes familles de polyphénols contribuent vraisemblablement de manière très importante à l'effet anti-oxydant et anti-glycation des protéines (M. Rudnicki et al, 2007) des parties aériennes de *P. edulis.*

Un effet anti-hypotenseur d'un extrait méthanolique d'écorces de fruits de *P. edulis* ainsi qu'un effet hypocholestémiant d'un extrait de graines délipidées riches en fibres ont également été mis en évidence. Effet anti-tumoral de décoction de fruit via l'inhibition de métalloprotéinases matricielles (MMP2 et MMP9) impliquées dans l'invasion tumorale, les métastases et l'angiogénèse.

### Usage Dermo-cosmétiques

Usage cutané des feuilles de *P. foetida* au Brésil pour traiter les maladies cutanées d'origine inflammatoire. A Maurice et Rodrigues les décoctions de feuilles de *P. suberosa* sont utilisées en bain pour traiter les maladies de peau.

### DESCRIPTION DE L'INVENTION

La Demanderesse a découvert que les extraits lipidiques de graines de *Passiflora incarnata* et/ou *edulis* présentent des propriétés cosmétiques et dermatologiques jamais décrites jusqu'à présent. En particulier, c'est la première fois que de tels extraits lipidiques de passiflore sont utilisés en tant que tels, pour leurs propriétés spécifiques.

L'invention a pour objet une composition comprenant un extrait lipidique de graines de passiflore, éventuellement en association avec un excipient approprié. La composition est avantageusement cosmétique, pharmaceutique, dermatologique, nutraceutique. Ladite composition est de préférence formulée pour être administrée par voie topique externe ou orale.

L'invention a pour objet un extrait lipidique de graines de Passiflores, *Passiflora incarnata* et/ou *Passiflora edulis,* préférentiellement de *P. edulis,* caractérisé en ce que ledit extrait lipidique est une huile de graines de Passiflores concentrée en sa fraction insaponifiable, contenant de 3% à 100% en poids, avantageusement 4% à 100% en poids, d'insaponifiables par rapport au poids total de l'extrait.

L'huile de graines de Passiflores concentrée en sa fraction insaponifiable contient les acides gras de l'huile originale et la répartition en acides gras de l'huile de passiflore concentrée est identique à celle de l'huile de passiflore avant concentration. De même, les composés insaponifiables et leur répartition respective sont identiques dans l'huile de départ et l'huile concentrée. Par contre, l'huile est concentrée en sa fraction insaponifiable, en particulier elle comprend plus de 3% en poids d'insaponifiables, par rapport au poids total de l'huile, avantageusement plus de 4% en poids d'insaponifiables, plus avantageusement plus de 4,5% en poids d'insaponifiables.

L'huile de graines de Passiflores concentrée en sa fraction insaponifiable comprend avantageusement des acides gras ayant de 12 à 22, plus avantageusement de 14 à 20, atomes de carbones. Ces acides gras peuvent être saturés, mono-insaturés ou polyinsaturés.

Un exemple de caractéristiques de l'huile brute de *Passiflora edulis* est donné dans le tableau 1 suivant :

**Tableau 1: exemple de caractéristiques d'huile de Passiflore brute**

| Coupe grasse (% en poids par rapport au poids total de l'huile) | |
|---|---|
| C14 (acide myristique) | ≤ 1,0 |
| C16 (acide palmitique) | 5,0 - 15,0 |
| C16' (acide 5-hexadécénoïque) | ≤ 1,0 |
| C18 (acide stéarique) | ≤ 5,0 |
| C18' (acide oléique) | 10,0 - 20,0 |
| C18" (acide linoléique) | 60,0 - 80,0 |
| C18"' (acide α-linolénique) | < 1,0 |
| C20 (acide arachidique) | ≤ 1,0 |
| C20' (acide eicasénoïque) | ≤ 1,0 |
| C22 (acide béhénique) | ≤ 1,0 |
| Teneur en Tocophérols (g/100g) | 0,001 - 0,5 |
| Teneur en Tocotriénols (g/100g) | 0,01 - 1,0 |
| Teneur en Stérols (g/100g) | 0,1 - 2,0 |
| Teneur en Squalène (g/100 g) | 0,05 - 1,0 |
| Teneur totale en Insaponifiables (g/100 g) | 0,3 - 2,0 |

La fraction insaponifiable est avantageusement majoritairement composée de tocophérols, de tocotriénols et de stérols. On retrouve également des squalènes.

Dans la fraction insaponifiable, la teneur en tocophérols varie avantageusement de 0,1% à 3% en poids, plus avantageusement de 0,5% à 3% en poids, encore plus avantageusement de 1% à 3% en poids, de tocophérols, par rapport au poids total de la fraction insaponifiable. Parmi les tocophérols, on retrouve avantageusement l'α-tocophérol, le β-tocophérol, le δ-tocophérol et le γ-tocophérol. Ces tocophérols constituent avantageusement de 60% à 100% en poids, plus avantageusement de 80% à 100% en poids, du poids total des tocophérols.

Dans la fraction insaponifiable, la teneur en tocotriénols varie avantageusement de 5% à 25% en poids, plus avantageusement de 8% à 25% en poids, encore plus avantageusement de 10% à 20% en poids, de tocotriénols, par rapport au poids total de la fraction insaponifiable. Parmi les tocotriénols, on retrouve avantageusement l'α-tocotriénol, le β- tocotriénol, le δ- tocotriénol. Ces tocotriénols constituent avantageusement de 60% à 100% en poids, plus avantageusement de 80% à 100% en poids, du poids total des tocotriénols.

Dans la fraction insaponifiable, la teneur en stérols varie avantageusement de 30% à 60% en poids, plus avantageusement de 35% à 55% en poids, plus avantageusement de 40% à50% en poids, de stérols par rapport au poids total de la fraction insaponifiable. Parmi les stérols, on retrouve avantageusement le campéstérol, le stigmastérol, le β-sitostérol et le Δ7-stigmastérol. Ces stérols constituent avantageusement de 60% à 100% en poids, plus avantageusement de 70% à 90% en poids, du poids total de la fraction insaponifiable.

Dans la fraction insaponifiable, la teneur en squalènes varie avantageusement de 10% à 35% en poids, plus avantageusement de 15% à 30% en poids, encore plus avantageusement de 15% à 25% en poids, de squalène, par rapport au poids total de la fraction insaponifiable.

La fraction insaponifiable comprend donc avantageusement des tocophérols, des tocotriénols, des stérols et des squalènes, de préférence dans les teneurs spécifiées auparavant. La fraction insaponifiable peut également comprendre d'autres insaponifiables non identifiés. Avantageusement, la teneur en ses insaponifiables non identifiés est inférieure à 30% en poids, par rapport au poids total de la fraction insaponifiable, plus avantageusement de 0% à 25% en poids.

Selon une première variante de l'invention, l'extrait lipidique est une huile de graines de Passiflores concentrée en sa fraction insaponifiable. Elle comprend avantageusement de 3 à 15% en poids d'insaponifiables, par rapport au poids total se de l'huile. Elle présente avantageusement les spécifications suivantes :

**Tableau 2: caractéristiques d'huile de Passiflore concentrée**

| Coupe grasse (% en poids par rapport au poids total de l'huile) | |
|---|---|
| C14 (acide myristique) | ≤ 1,0 |
| C16 (acide palmitique) | 5,0 - 15,0 |
| C16' (acide 5-hexadécénoïque) | ≤ 1,0 |
| C18 (acide stéarique) | ≤ 5,0 |
| C18' (acide oléique) | 10,0 - 20,0 |
| C18" (acide linoléique) | 60,0 - 80,0 |
| C18"' (acide α-linolénique) | < 1,0 |
| C20 (acide arachidique) | ≤ 1,0 |
| C20' (acide eicasénoïque) | ≤ 1,0 |
| C22 (acide béhénique) | ≤ 1,0 |
| Teneur totale en Insaponifiables (g/100 g) | 3,0-15,0 |

La fraction insaponifiable est telle que décrite précédemment.

Selon une deuxième variante de l'invention, l'extrait lipidique est la fraction insaponifiable. Cette fraction insaponifiable est telle que décrit précédemment

L'extrait lipidique est avantageusement obtenu par un procédé comprenant les étapes successives suivantes :
a) distillation moléculaire d'une huile brute ou raffinée de passiflore ;
b) le cas échéant, extraction de l'insaponifiable ;
c) récupération de l'huile concentrée en insaponifiable obtenue suite à l'étape a) ou de l'insaponifiable obtenu suite à l'étape b).

Lorsque l'on désire récupérer l'huile enrichie en insaponifiable, l'étape b) n'est pas mise en oeuvre.

Lorsque l'on désire récupérer la fraction insaponifiable, l'étape b) est mise en oeuvre. Cette étape b) comprend avantageusement les étapes successives suivantes :
i. saponification de l'huile de passiflore concentrée en sa fraction insaponifiable obtenue suite à l'étape a)
ii. puis extraction de l'insaponifiable à l'aide d'un solvant approprié.
Ce procédé est décrit par la suite.

L'invention a également pour objet un procédé de préparation d'un extrait selon l'invention comprenant les étapes successives suivantes :
a) distillation moléculaire d'une huile brute ou raffinée de passiflore ;
b) le cas échéant, extraction de l'insaponifiable ;
c) récupération de l'huile concentrée en insaponifiable obtenue suite à l'étape a) ou de l'insaponifiable obtenu suite à l'étape b).

A partir de graines de passiflore, le procédé comprend une première étape d'extraction de l'huile par des technologies connues de l'Homme de l'art comme par exemple la pression, l'extraction par solvants, sous pression supercritique et plus particulièrement par pression ou sous pression supercritique et préférentiellement par pression. L'huile peut être raffinée ou non par les technologies connues de l'Homme de l'art et préférentiellement non raffinée. Cette huile est ensuite soumise à une distillation moléculaire pour conduire à une huile concentrée en sa fraction insaponifiable (ou concentrât).

Les huiles peuvent être extraites par plusieurs procédés :
- extraction physique telle que la pression à froid sur presse mécanique, la pression sur extrudeuse bi-vis ;
- extraction chimique à l'aide de solvants organiques (alcanes aliphatiques, alcools, solvants chlorés, solvants fluorés) ;
- extraction en milieu supercritique, à l'aide du dioxyde de carbone par exemple, seul et/ou avec des co-solvants.

Pour l'extraction d'huile brute de passiflore, la pression à froid sur presse mécanique sera privilégiée.

L'huile brute de Passiflore peut être raffinée selon des procédés connus de l'homme de métier tels que le raffinage physique (dégommage à l'eau, désacidification par désodorisation à haute température) et le raffinage chimique (démucilagination à l'eau ou traitement acide afin d'éliminer les phospholipides, neutralisation des acides gras libres à l'aide d'une solution basique, décoloration, frigelisation et désodorisation).

L'huile brute de passiflore obtenue précédemment est avantageusement concentrée en sa fraction insaponifiable par un procédé de distillation moléculaire.

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques ou terpéniques, stérols, tocophérols, tocotriénols, les pigments caroténoïdes et xanthophylles.

Cette étape de distillation moléculaire est de préférence réalisée en utilisant un dispositif choisi parmi les distillateurs moléculaires de type centrifuge et les dispositifs moléculaires de type à film raclé.

Les distillateurs moléculaires de type centrifuge sont connus de l'homme de métier. Par exemple, la demande EP 493 144 décrit un distillateur moléculaire de ce type. D'une manière générale, le produit à distiller est étalé en couche mince sur la surface chauffée (surface chaude) d'un rotor conique tournant à grande vitesse. L'enceinte de distillation est placée sous vide. Dans ces conditions, il y a évaporation et non pas ébullition, depuis la surface chaude, des constituants de l'huile tels que les insaponifiables, l'avantage étant que l'huile et ses constituants, notamment les insaponifiables (ces produits étant réputés fragiles), ne sont pas dégradés au cours de l'évaporation.

Les distillateurs moléculaires de type à film raclé sont également connus de l'homme du métier. D'une manière générale, ils comprennent une chambre de distillation dotée d'un racleur tournant, permettant l'étalement en continu sur la surface d'évaporation (surface chaude) des produits à distiller. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile, etc.). La récupération des résidus et des distillats dans des ballons en verre se fait par écoulement gravitationnel.

A l'issue de l'étape de fractionnement, la fraction distillée riche en insaponifiables représente avantageusement 3 à 15 % en poids de l'huile de départ, et la fraction distillée riche en triglycérides représente avantageusement 85 à 97 % en poids de l'huile de départ.

Il a en outre été vérifié que ce procédé n'entraînait aucune modification chimique ou altération des composés de l'insaponifiable, et que les fractions fortement insaturées étaient préservées. Par conséquent, la répartition en acides gras de l'huile de passiflore concentrée est identique à celle de l'huile de passiflore avant concentration.

Suite à cette étape de distillation moléculaire, le produit obtenu peut-être éventuellement désodorisé et/ou décoloré par des procédés connus de l'Homme de l'art.

L'huile de passiflore concentrée en sa fraction insaponifiable ainsi obtenue présente les caractéristiques décrites précédemment.

Lorsque l'on désire récupérer l'huile enrichie en insaponifiable, l'étape b) n'est pas mise en oeuvre.

Lorsque l'on désire récupérer la fraction insaponifiable, l'étape b) est mise en oeuvre. Cette étape comprend avantageusement les étapes successives suivantes :
i. saponification de l'huile de passiflore concentrée en sa fraction insaponifiable obtenue suite à l'étape a)
ii. puis extraction de l'insaponifiable à l'aide d'un solvant approprié.

L'insaponifiable d'huile de passiflore peut être obtenu par des procédés connus de l'homme de métier. Par exemple, il peut être obtenu en effectuant une saponification sur l'huile de passiflore concentrée en sa fraction insaponifiable, puis en extrayant cet insaponifiable à l'aide d'un solvant approprié. Cet extrait est ensuite lavé jusqu'à élimination complète des savons puis le solvant est évaporé. Enfin l'insaponifiable subit avantageusement une désodorisation à la vapeur d'eau puis stripping à l'azote afin d'éliminer les traces de solvant.

L'insaponifiable d'huile de passiflore ainsi obtenu présente avantageusement les caractéristiques décrites précédemment.

Dans le cadre de l'invention, l'extrait lipidique de graines de passiflore est lui-même choisi dans le groupe constitué par une huile concentrée en sa fraction insaponifiable, une fraction insaponifiable, la fraction insaponifiable ayant les spécifications données précédemment:
L'invention a aussi pour objet une composition comprenant, en tant qu'actif, un extrait lipidique de graines de Passiflores selon l'invention et un excipient approprié.

La composition comprend avantageusement de 0,01% à 20% en poids dudit extrait lipidique, par rapport au poids total de la composition.

L'extrait est avantageusement utilisé en tant qu'agent actif dans une composition telle qu'une composition cosmétique, dermatologique ou pharmaceutique, qui peut comprendre un ou plusieurs excipients appropriés. La composition peut en outre comprendre au moins un autre composé actif en plus de l'extrait lipidique de passiflore. Cet autre composé peut être choisi parmi tous les composés et leurs équivalents fonctionnels, énoncés ci-dessous.

Cet autre composé peut être en particulier choisi parmi des actifs classiquement utilisés en dermatologie ou cosmétique tels que les émollients, les actifs hydratants, les activateurs de la synthèse de kératine, les kératorégulateurs, les kératolytiques, les agents restructurant de la barrière cutanée (activateurs de la synthèse des lipides cutanés), des agonistes PPARs (ou Peroxysome Proliferator Activated Receptor), les agonistes RXR ou LXR, les agents sébo-régulateurs, les agents anti-irritants, les agents apaisants, les agents anti-inflammatoires, les agents anti-oxydants et les agents anti-âge, les agents dépigmentants ou hypodépigmentants, les agents pigmentants, les agents lipolytiques ou inhibiteurs de la lipogénèse ou encore les agents anti-cellulite ou amincissants, les filtres et écrans solaires minéraux ou organiques, les composés antifongiques, les conservateurs, les agents anti-bactériens, les pré et probiotiques, les antibiotiques, les immuno-modulateurs.

Plus particulièrement, les agents cicatrisants et/ou restructurants de la barrière cutanée pouvant être utilisés en association sont avantageusement le panthénol (vitamine B5), l'arabinogalactane, l'oxyde de zinc, les céramides, le cholestérol, le squalane et les phospholipides.

Les agents sébo-régulateurs pouvant être utilisés en association sont avantageusement choisis dans le groupe constitué par les inhibiteurs de 5-alpha-réductase. Le zinc (et les dérivés de zinc tels que ses sels gluconate, salicylate et acide pyroglutamique) et la spironolactone, présentent aussi une activité sébo-suppresseur. D'autres séborégulateurs d'origine lipidique agissant sur la qualité du sébum, comme l'acide linoléique présentent également un intérêt.

L'agent anti-inflammatoire et/ou anti-irritant et/ou apaisant peut être l'arabinogalactane.

Les actifs protecteurs solaires pouvant être utilisés en association sont avantageusement des filtres et écrans solaires UVB et/ou UVA, tels les écrans ou filtres minéraux et/ou organiques connus de l'Homme du métier qui adaptera leur choix et leurs concentrations en fonction du degré de protection recherché.

Les conservateurs pouvant être utilisés en association sont par exemple ceux généralement utilisés en cosmétique, les molécules à activité anti-bactérienne (pseudo-conservateurs) tels que les dérivés capryliques comme par exemple le capryloyl glycine et le glycéryl caprylate ; l'hexanediol, le sodium levulinate, et les dérivés de zinc et de cuivre (gluconate et PCA).

Parmi les actifs recommandés en association avec l'extrait selon l'invention, on peut citer les extraits végétaux, en particulier :
- les huiles végétales telles que l'huile de soja et/ou l'huile de colza, l'huile d'avocat (WO2004/012496, WO2004/012752, WO2004/016106, WO2007/057439), l'huile de lupin et avantageusement l'huile de lupin blanc doux (WO98/47479), ou un mélange de ces huiles ;
- l'oléodistillat ou les concentrats d'huile végétale ou animale, notamment de tournesol, plus avantageusement des concentrats de tournesol linoléiques, tels que l'huile de tournesol concentrée en insaponifiables (Soline® - WO2001/21150), commercialisée par les Laboratoires Expanscience, les huiles concentrées en insaponifiables du type huile d'avocat, de colza, de maïs utiles notamment pour leur activité hydratante et/ou émolliente, cicatrisante et/ou restructurante de la barrière cutanée, anti-inflammatoire et/ou anti-irritante et/ou apaisante ;
- Les insaponifiables de végétaux ou d'huile végétale, avantageusement des furanes d'avocat (Avocadofurane®), pouvant être obtenus par le procédé décrit dans la demande internationale WO 01/21605, les insaponifiables d'Avocat et/ou de Soja, plus particulièrement un mélange d'insaponifiables d'Avocat furaniques et d'insaponifiables de soja, avantageusement dans un rapport respectif d'environ 1/3 - 2/3 (tel que Piasclédine®), les insaponifiables de soja (tels qu'obtenus selon le procédé décrit dans le demande internationale WO 01/51596), les insaponifiables stéroliques (typiquement des insaponifiables dont la teneur en stérols, en méthylstérols et en alcool triterpéniques est comprise entre 20 et 95% en poids, de préférence 45-65% en poids, par rapport au poids total de l'insaponifiable), les phytostérols, les esters de stérols et les dérivés vitaminiques, utiles notamment pour leur activité cicatrisante et/ou restructurante de la barrière cutanée, anti-âge, anti-inflammatoire ;
- Les peptides ou complexes d'acides aminés végétaux, en particulier les d'avocat (tel que ceux décrits dans la demande internationale WO2005/105123), les peptides de lupin (tel que ceux décrits dans la demande internationale WO2005/102259), les peptides de quinoa (tel que ceux décrits dans la demande internationale WO2008/080974), les peptides de maca (tel que ceux décrits dans la demande internationale WO2004/112742), les peptides de soja fermenté ou non, les peptides de riz (tel que ceux décrits dans la demande internationale WO2008/009709), utiles notamment pour leur activité hydratante et/ou émolliente (avocat), kératorégulatrice (lupin, quinoa), cicatrisante et/ou restructurante de la barrière (maca, quinoa, soja), anti-inflammatoire et/ou anti-irritante et/ou apaisante (lupin, quinoa), antioxydante (avocat), anti-âge (lupin, maca), pigmentante (riz), les peptides de schizandra (tel que ceux décrits dans la demande de brevet FR 0955344), l'extrait de graines d'*Acacia macrostachya* (tels que celui décrit dans la demande WO 2011/064402), l'extrait de graines de *Vigna unguiculata* (tels que celui décrit dans la demande WO 2011/064401) ; les extraits peptidiques et osidiques de graines de passiflore (tel que ceux décrits dans la demande de brevet FR1262234)
- Les sucres végétaux, en particulier les sucres d'avocat (tels que ceux décrits dans la demande WO2005/115421), utiles notamment pour leur propriété kératorégulatrice, cicatrisante et/ou restructurante de la barrière cutanée, anti-inflammatoire et/ou irritante et/ou apaisante ;
- Le butyle avocadate (5 alpha Avocuta®), inhibiteur de la 5-alpha réductase (WO 01/52837 et WO 02/06205) et typiquement, régulateur de la sécrétion séborrhée se trouvant augmentée dans l'acné et les pellicules ;
- Les extraits riches en polyphénols, et plus particulièrement les extraits de fruits d'avocat (tels que ceux décrits dans la demande FR 1 061 055), les extraits de feuilles de maca (tels que ceux décrits dans la demande FR 1 061 047), et les extraits de parties aériennes de *Gynandropsis gynandra* (tels que ceux décrits dans la demande FR 1 061 051),
- Le lupéol (FR 2 8 22 821, FR 2 857 596) utile notamment pour favoriser la cicatrisation ;
- Un beurre de Cupuaçu, particulièrement apprécié pour ses propriétés hydratantes.

Parmi les actifs recommandés en association avec l'extrait selon l'invention, on peut citer les oxazolines, en particulier celles choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline (de préférence la 2-undécyl-4,4-diméthyl-1,3-oxazoline, appelée OX-100 ou Cyclocéramide® ; WO2004050052, WO2004050079, et WO2004112741). Elles sont particulièrement utiles pour leur activité anti-inflammatoire et/ou anti-irritante et/ou apaisante, antioxydante, dépigmentant, immunomodulatrice.

Toutes ces associations comprennent au moins un autre composé actif, en plus de l'extrait de graines de Passiflore, et peuvent comprendre deux, trois, quatre ou plus de composés actifs tels que décrits précédemment.

La composition selon l'invention peut être formulée sous la forme de différentes préparations adaptées à une administration topique, à une administration orale, rectale, vaginale, nasale, auriculaire ou bronchique, ainsi qu'à une administration parentérale. Avantageusement, la composition selon l'invention est formulée sous la forme d'une préparation adaptée à une administration topique ou à une administration orale.

Selon une première variante, les différentes préparations sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

La composition comprenant un extrait de graines de passiflores ayant les spécifications indiquées est particulièrement destinée à une utilisation cosmétique, pharmaceutique, dermatologique, nutraceutique.

Dans le cadre d'une utilisation cosmétique, pharmaceutique, ou dermatologique, la composition sera avantageusement formulée sous la forme d'une préparation adaptée à une administration topique. La composition comprenant un extrait lipidique de graines de passiflore lui-même choisi dans le groupe constitué par une huile concentrée en sa fraction insaponifiable, un insaponifiable, est particulièrement destinée à une utilisation cosmétique, pharmaceutique, ou dermatologique.

Dans le cadre d'une utilisation alimentaire, à visée nutritive ou cosmétique (« cosmet-food »), la composition sera avantageusement formulée sous la forme d'une préparation adaptée à une administration orale.

L'invention a également pour objet un extrait selon l'invention ou une composition selon l'invention pour son utilisation en tant que, ou dans une, composition dermatologique, pharmaceutique ou en tant que, ou dans un, aliment fonctionnel.

Un aliment fonctionnel est un aliment conventionnel, ou qui en a l'apparence, qui fait partie de l'alimentation normale, et qui a pour caractéristique de procurer des effets physiologiques bénéfiques dépassant ses fonctions nutritionnelles habituelles ou de réduire le risque de maladies chroniques.

On a démontré que l'extrait selon l'invention, et en particulier une huile de *Passiflore edulis* concentrée en sa fraction insaponifiable,
- Contribue à protéger le derme des dommages oxydatifs
- Augmente la biosynthèse d'acide hyaluronique et donc favorise l'hydratation du derme et son élasticité
- contribue à augmenter la fibrillogénèse et le remodelage de la matrice extracellulaire du derme pour permettre une meilleure cohésion et élasticité du derme
- augmente la prolifération de fibroblastes humains normaux
- diminue les forces contractiles générées par les fibroblastes de vergeture rouge
- favorise la lipolyse adipocytaire
- réprime l'expression de gènes impliqués dans la mélanogénèse

Avantageusement, la composition ou l'extrait selon la présente invention est utilisée dans la prévention et/ou le traitement des troubles ou pathologies de la peau et/ou des muqueuses et/ou des phanères. En particulier, la composition ou l'extrait selon l'invention est utilisée pour stimuler, restaurer ou réguler le métabolisme des cellules de la peau et des muqueuses et/ou dans la prévention et/ou le traitement des troubles liés au tissu dermique.

En particulier, l'extrait selon l'invention peut être utilisé pour une ou plusieurs des indications suivantes :
- en tant qu'agent anti-âge
- en tant qu'agent cicatrisant ;
- pour prévenir une altération de, et/ou maintenir l'homéostasie de la peau ou des muqueuses ;
- en tant qu'agent antioxydant
- en tant qu'agent anti-inflammatoire ;
- en tant qu'agent amincissant et/ou anti-cellulite
- pour prévenir ou traiter les vergetures de la peau
- en tant qu'agent dépigmentant

Plus particulièrement, la composition ou l'extrait selon l'invention peuvent être utilisés pour prévenir ou retarder le vieillissement cutané prématuré, en particulier photo-induit. Ils sont donc utiles dans une crème ou autre formulation visant prévenir, réduire et/ou traiter les rides, les ridules ou une altération du microrelief.
De par son action sur la prolifération des fibroblastes et sur le collagène notamment, la composition ou l'extrait selon l'invention peuvent être utilisés pour renforcer les propriétés mécaniques de la peau et des muqueuses, en particulier pour lutter contre la peau flétrie, molle, affaissée et/ou amincie, et/ou renforcer et/ou restaurer l'élasticité ou la fermeté de la peau.

La composition ou l'extrait selon l'invention peuvent également être utilisés pour la prévention et/ou le traitement des altérations du tissu adipeux.

En particulier, la composition ou l'extrait selon l'invention est destiné à la prévention et/ou au traitement des réactions ou pathologies allergiques, inflammatoires, irritatives ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

La composition ou l'extrait selon l'invention peuvent également être utilisés pour favoriser la cicatrisation.
La composition ou l'extrait selon l'invention peuvent ainsi être utilisés dans la prévention et/ou le traitement des pathologies ou conditions choisies dans le groupe constitué par les cicatrices superficielles, les lèvres fragiles et les cheilites, les vergetures, la peau après piqûres, les abrasions de la peau, les boutons et/ou les croûtes cutanés, et les peaux fragiles et sensibles. Ainsi, la composition ou l'extrait selon l'invention est particulièrement adapté dans la prévention ou le traitement de vergetures de la peau.

Par l'expression « prévention des vergetures de la peau », on entend selon la présente invention une action permettant d'éviter ou à tout le moins de réduire la formation de vergetures, c'est-à-dire leur longueur, largeur et/ou profondeur, dans le cadre d'un traitement cosmétique ou dermatologique, par application de la composition, avant et au cours d'un évènement connu comme pouvant provoquer l'apparition de vergetures, tel qu'une grossesse. Par l'expression « traitement des vergetures de la peau », on entend selon la présente invention une action permettant de faire régresser, c'est-à-dire de régresser, c'est-à-dire de résorber, dans le cadre d'un traitement cosmétique ou dermatologique, de manière visible et mesurable, des vergetures déjà formées, c'est-à-dire leur longueur, largeur et/ou profondeur.

Ainsi, la composition utilisée selon l'invention peut être appliquée sur des zones de la peau susceptibles de former des vergetures, comprenant des vergetures en cours de formation ou même comprenant des vergetures déjà formées.

Avantageusement, la composition ou l'extrait selon l'invention peut être utilisé(e) pour la prévention et/ou le traitement des réactions, troubles ou pathologies :
- de la peau, telles que l'acné, la rosacée ou érythrocouperose, le psoriasis, les troubles vasculaires, la dermite du siège, la dermatite atopique, l'eczéma, la dermatite de contact, la dermatite irritative, la dermatite allergique, la dermite séborrhéique (croûte de lait), le psoriasis, la peau sensible, la peau réactive, la peau sèche (xérose), la peau déshydratée, la peau avec rougeur, l'érythème cutané, la peau âgée ou photoâgée, la peau photosensibilisée, la peau pigmentée (mélasma, pigmentation post-inflammatoire...), la peau dépigmentée (vitiligo), la peau avec cellulite, la peau relâchée, la peau avec vergetures, les dartres, les gerçures, les piqûres, les crevasses en particulier des seins, les coups de soleil, les inflammations dus aux rayons de toutes sortes, les irritations par agents chimiques, physiques (par exemple contrainte de tension pour les femmes enceintes), bactériologiques, fongiques ou viraux, parasitaires (poux, gale, teigne, acariens, dermatophytes), radiologiques ou par déficit de l'immunité innée (peptides antimicrobiens) ou acquise (cellulaire, humorales, cytokines), et/ou
- des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales mâles ou femelles externes ou internes et/ou
- des phanères tels que les ongles (ongles cassants, fragiles ...) et des cheveux (alopécie, pellicules, hirsutisme, dermites séborrhéiques, folliculites) immatures, normaux ou matures, présentant en particulier des troubles du cuir chevelu tels que les alopécies (ou pelade) androgénétiques, aiguës, localisées, cicatricielles, congénitales, occipitales du nourrisson, aerata, dues à la chimiothérapie/radiothérapie ou encore l'effluvium télogène, l'effluvium anagène, la dystrophie pilaire, la trichotillomanie, la teigne ou les pellicules grasses ou sèches.

L'invention concerne également un procédé de soin cosmétique de la peau et/ou des phanères et/ou des muqueuses, en vue d'améliorer leur état et/ou leur aspect, avantageusement en vue d'améliorer la fermeté, l'élasticité ou la tonicité de la peau comprenant l'administration, par voie orale ou topique, d'un extrait selon l'invention ou d'une composition selon l'invention.

L'invention a aussi pour objet une méthode de traitement cosmétique des peaux sèches, avec sensation de tiraillement, comprenant l'administration, par voie orale ou topique, d'un extrait selon l'invention ou d'une composition selon l'invention..

L'invention a aussi pour objet une méthode de traitement cosmétique pour remodeler la silhouette, limiter l'effet « peau d'orange » comprenant l'administration, par voie orale ou topique, d'un extrait selon l'invention ou d'une composition selon l'invention.

L'invention a enfin pour objet un aliment fonctionnel comprenant un extrait lipidique tel que défini précédemment. L'aliment fonctionnel peut être choisi parmi :
1) Les produits laitiers : tels que les fromages, le beurre, le lait et autres breuvages lactés, mélanges et pâtes à tartiner à base de produits lactés, crèmes glacées et yaourts ;
2) Les produits à base de graisse tels que les margarines, pâtes à tartiner, mayonnaises, matières grasses pour cuisson, huiles à frire et vinaigrettes ;
3) Les produits à base de céréales composés de graines tels que le pain et les pâtes, que ces aliments soient cuisinés, cuits au four ou transformés.
4) Les confiseries tels que le chocolat, les bonbons, les chewing gums, les desserts, les nappages, les sorbets, les glaçages, et autres garnitures ;
5) Les boissons alcoolisées ou non, y compris les sodas et autres boissons non alcoolisées, jus de fruits, compléments diététiques, substituts de repas sous forme de breuvage comme ceux vendus sous la marque Boost™ and Ensure™ et ;
6) Les produits divers comme les oeufs, les aliments transformés comme les soupes, les sauces toutes prêtes pour pâtes, des plats préparés et autre produits du même genre.

La composition ou l'extrait de la présente invention peut être incorporée directement et sans autre modification dans la nourriture, les nutraceutiques, les produits diététiques notamment hyperprotéinés ou les breuvages et ce grâce à des techniques comme le mixage, l'infusion, l'injection, le mélange, l'absorption, le pétrissage et la pulvérisation.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, ou vétérinaire adapté à un patient ou à un animal comme par exemple l'âge ou le poids corporel du patient ou de l'animal, la gravité de son état général, la tolérance au traitement, les effets secondaires constatés, le type de peau.

### REFERENCES BIBLIOGRAPHIQUES

Georges PIOMBO et al., OCL vol. 13 no. 2-3 mars-juin 2006
Martina RUDNICKI et al., Food Chemistry 100 (2007) 719-724
David CHASSAGNE and Jean CROUZET, Phytochemistry, vol. 49, no. 3 pp 757-759, 1998
Ezio BOMBARDELLI et al., Phytochemistry, 1975, Vol. 14, pp 2661-2665
Shoko SANO et al., Agric. Food Chem., 2011, 59, 6209-6213
Kamaldeep DHAWAN et al., Journal of Ethnopharmacology 94 (2004) 1-23
Zerark et al, microchemical Journal 96(2010), 86-91
R. de V. Vieira Lopes et al., Eur. J. Lipid. Sci. Technol. 2010, 112, 1253-1262
Seigler et al., Phytochemistry 60 (2012), 873-882

### Exemple 1 : Concentrât selon l'invention

Un exemple de concentrât selon l'invention est une huile brute de *P. Edulis* concentrée en sa fraction insaponifiable. L'extrait est préparé par distillation moléculaire.

Cet extrait présente la répartition massique suivante :

**Tableau 3**

| **Coupe grasse** (% en poids par rapport au poids total de l'huile) | |
|---|---|
| C14 | 0,1 |
| C16 | 13,3 |
| C16' | 0,4 |
| C18 | 2,5 |
| C18' | 15 |
| C18" | 67,5 |
| C18 »' | 0,4 |
| C20 | 0,1 |
| C20' | 0,1 |
| C22 | 0 |
| C22' | 0 |
| Teneur en Tocophérols (g/100g) | 0,09 |
| Teneur en Tocotriénols (g/100g) | 0,57 |
| Teneur en Stérols (g/100g) | 2,13 |
| Teneur en Squalène (g/100 g) | 0,87 |
| Teneur totale en Insaponifiables (g/100 g) | 4,7 |

Les tocophérols présentent la répartition massique suivante :

**Tableau 4**

| | |
|---|---|
| % α-tocophérol | 7,5 |
| % β-tocophérol | 3,5 |
| % δ-tocophérol | 17 |
| % γ-tocophérol | 72 |

Les tocotriénols présentent la répartition massique suivante :

**Tableau 5**

| | |
|---|---|
| % α-tocotriénol | 2 |
| % δ-tocotriénol | 58 |
| % γ-tocotriénol | 40 |

Les stérols présentent la répartition massique suivante :

**Tableau 6**

| | |
|---|---|
| % campestérol | 9,5 |
| % stigmastérol | 24 |
| % β-sitostérol | 33 |
| % Δ7-stigmastérol | 3,5 |
| % non identifiés | 30 |

### Exemple 2 : Compositions pour application par voie topique

Plusieurs compositions pour application par voie topique sont présentées ci-dessous. L'extrait lipidique de graines de passiflore lui-même choisi dans le groupe constitué par une huile concentrée en sa fraction insaponifiable, un insaponifiable, peut être incorporé à divers produits cosmétiques, tels que des eaux nettoyantes, des émulsions huile dans eau, des émulsions eau dans huile, des huiles, des laits, des lotions, des shampooings, des produits moussants et sprays, dont les compositions sont présentées ci-dessous à titre d'exemples.

**EAU NETTOYANTE HYDRATANTE :**

| **Matière première / Nom commercial ou Nom INCI** | **%** |
|---|---|
| EAU PURIFIÉE | QSP 100 % |
| BIOSACCHARID GUM | De 1 à 5 % |
| BUTYLENE GLYCOL | De 1 à 5 % |
| ACIDE HYALURONIQUE | De 0 à 5 % |
| *CONCENTRAT DE PASSIFLORE* | De 0,001 à 20 % |
| CONSERVATEURS | De 0 à 1 % |
| ACIDE CITRIQUE MONOHYDRATE | De 0 à 1 % |
| TRIMETHAMINE | De 0 à 1 % |

**EAU NETTOYANTE PEAU SENSIBLE :**

| **Matière première / Nom commercial ou Nom INCI** | **%** |
|---|---|
| CAPRYLOYL GLYCINE | De 0 à 1 % |
| LESSIVE SOUDE | De 0 à 1 % |
| SEQUESTRANT | De 0 à 1 % |
| BUTYLENE GLYCOL | De 1 à 5 % |
| BETA CAROTENE | De 0 à 2 % |
| *CONCENTRAT DE PASSIFLORE* | De 0,001 à 20 % |
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1 % |
| POLOXAMER 184 | De 1 à 5 % |

**EMULSION ANTI-AGE :**

| **Matière première / Nom commercial ou Nom INCI** | **%** |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1% |
| VITAMINE C | De 0 à 5% |
| *CONCENTRAT DE PASSIFLORE* | De 0,01 à 20 % |
| ISONONYL ISONONANOATE | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

**EMULSION RESTRUCTURANTE :**

| **Matière première / Nom commercial ou Nom INCI** | **%** |
|---|---|
| HYDROGENATED POLYDECENE | De 5 à 20 % |
| LAURYLGLUCOSIDE-GLYSTEARATE | De 1 à 5 % |
| DICAPRYLYL CARBONATE | De 1 à 5 % |
| GLYCEROL | De 5 à 20 % |
| CARBOPOL | De 0 à 1 % |
| GOMME XANTHANE | De 0 à 1 % |
| ACIDE ASIATIQUE | De 0 à 1 % |
| VITAMINE B5 | De 0 à 5 % |
| *CONCENTRAT DE PASSIFLORE* | De 0,01 à 20 % |
| LESSIVE SOUDE | De 0 à 1 % |
| CONSERVATEURS | De 0 à 1 % |
| ACIDE CITRIQUE | De 0 à 1 % |
| EAU PURIFIÉE | QSP 100 % |

**HUILE AMINCISSANTE :**

| **Matière première / Nom commercial ou Nom INCI** | **%** |
|---|---|
| SOLUBILISANT | De 0 à 1 % |
| HUILE AMANDE DOUCE | De 5 à 20 % |
| CAPRYLATE / CAPRATE DE COPRAH | QSP 100 % |
| HUILE DE MACADAMIA RAFFINEE | De 5 à 20 % |
| CAPRYLO CAPRATE DE GLYCEROL | De 5 à 20 % |
| ALPHA BISABOLOL NAT | De 0 à 1 % |
| ALPHA TOCOPHEROL | De 0 à 1 % |
| EXTRAIT DE LIERRE | De 0 à 5 % |
| *CONCENTRAT DE PASSIFLORE* | De 0,01 à 20 % |
| CONSERVATEUR | De 0 à 1 % |
| ESTER | De 0 à 1 % |

**LAIT pour PEAU SECHE, ATOPIQUE :**

| **Matière première** / **Nom commercial ou Nom INCI** | **%** |
|---|---|
| HUILE AMANDE DOUCE | De 1 à 5 % |
| HUILE MAIS | De 1 à 5 % |
| ACIDE STEARIQUE | De 1 à 5 % |
| ALCOOL CETYLIQUE C16 C18 | De 0 à 1 % |
| ANTIMOUSSE 70414 | De 0 à 1 % |
| ALCOOL LAURIQUE 11OE | De 1 à 5 % |
| MONOLAURATE PEG 300 | De 0 à 1 % |
| MONOLEATE DE GLYCEROL | De 0 à 1 % |
| MONOSTEARATE DE GLYCEROL | De 1 à 5 % |
| VITAMINE B12 | De 0 à 5% |
| *CONCENTRAT DE PASSIFLORE* | De 0,1 à 120 % |
| CONSERVATEURS | De 0 à 1 % |
| ACIDE CITRIQUE | De 0 à 1 % |
| CITRATE TRISODIQUE | De 0 à 1 % |
| EAU PURIFIEE | QSP 100 % |
| PARFUM | De 0 à 1 % |
| HUILE ARACHIDE | De 1 à 5 % |
| HUILE PALMISTE HYDROGENEE | De 1 à 5 % |

**MOUSSANT :**

| **Matière première / Nom commercial ou Nom INCI** | % |
|---|---|
| EAU PURIFIÉE | QSP 100 % |
| LAUROAMPHOACETATE | De 5 à 20 % |
| COCOGLUCOSIDE | De 5 à 20 % |
| TENSIOACTIF 1 | De 5 à 20 % |
| TENSIOACTIF 2 | De 5 à 20 % |
| DISTEARATE DE PEG 6000 | De 1 à 5 % |
| CONSERVATEURS | De 1 à 5 % |
| *CONCENTRATION DE PASSIFLORE* | De 0,001 à 12 % |
| EXTRAIT CAMOMILLE | De 1 à 5 % |
| ACIDE CITRIQUE MONOHYDRATE | De 0 à 1 % |
| SEQUESTRANT | De 0 à 1 % |
| PARFUM | De 0 à 1 % |
| LESSIVE SOUDE | De 0 à 1 % |

**SPRAY APAISANT :**

| **Matière première / Nom commercial ou Nom INCI** | **%** |
|---|---|
| EAU PURIFIÉE | QSP 100 % |
| TRILAURETH-4 PHOSPHATE | De 1 à 5 % |
| DICAPRYLYL CARBONATE | De 1 à 5 % |
| BUTYLENE GLYCOL | De 1 à 5 % |
| ERYTHRITYL ESTER | De 1 à 5 % |
| HUILE VASELINE FLUIDE | De 1 à 5 % |
| BEURRE DE KARITE | De 0 à 1 % |
| HUILE VEGETALE | De 0 à 1 % |
| CONSERVATEURS | De 0 à 1 % |
| LYCOPENE | De 0 à 5 % |
| *CONCENTRAT DE PASSIFLORE* | De 0,01 à 20 % |
| LESSIVE SOUDE | De 0 à 1 % |
| PARFUM | De 0 à 1 % |
| GOMME XANTHANE | De 0 à 1 % |
| CARBOPOL | De 0 à 1 % |
| SEQUESTRANT | De 0 à 1 % |
| ACIDE CITRIQUE | De 0 à 1 % |

**CREME LAVANTE PURIFIANTE :**

| **Matière première / Nom commercial ou Nom INCI** | **%** |
|---|---|
| EAU PURIFIEE | QSP 100 % |
| ARLATONE | De 10 à 30 % |
| COCOGLUCOSIDE | De 5 à 20 % |
| HYDROXYPROPYL GUAR | De 1 à 5 % |
| CAPRYLOYL GLYCINE | De 0 à 2 % |
| CONSERVATEURS | De 0 à 2 % |
| PARFUM | De 0 à 1 % |
| ACIDE CITRIQUE | De 0 à 1 % |
| ZINC PCA | De 0 à 1 % |
| *CONCENTRAT DE PASSIFLORE* | De 0,001 à 20 % |

**EMULSION ANTI-ACNEIQUE :**

| **Matière première / Nom commercial ou Nom INCI** | **%** |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITANE | De 0 à 2 % |
| ALCOOL CETYLIQUE | De 0 à 2 % |
| ALCOOL DI-MALATE | De 5 à 20 % |
| VITAMINE E | De 0 à 1 % |
| VITAMINE B3 | De 0 à 5 % |
| ACIDE LINOLEIQUE | De 0 à 1 % |
| *CONCENTRAT DE PASSIFLORE* | De 0,01 à 20 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

**EMULSION ANTI-ROUGEURS :**

| **Matière première / Nom commercial ou Nom INCI** | **%** |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITANE | De 0 à 2 % |
| ALCOOL CETYLIQUE | De 0 à 2 % |
| ALCOOL DI-MALATE | De 5 à 20 % |
| ESCULOSIDE | De 0 à 2 % |
| SOPHORA JAPONICA | De 0 à 5 % |
| VITAMINE E | De 0 à 1 % |
| *CONCENTRAT DE PASSIFLORE* | De 0,01 à 20 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

**SOIN REPARATEUR :**

| **Matière première / Nom commercial ou Nom INCI** | **%** |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITANE | De 0 à 2 % |
| ALCOOL CETYLIQUE | De 0 à 2 % |
| ALCOOL DI-MALATE | De 5 à 20 % |
| VITAMINE E | De 0 à 1 % |
| COENZYME Q10 | De 0 à 2 % |
| CERAMIDE | De 0 à 5 % |
| *CONCENTRAT DE PASSIFLORE* | De 0,01 à 20 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

**EMULSION DEPIGMENTANTE :**

| **Matière première / Nom commercial ou Nom INCI** | **%** |
|---|---|
| ISONONYL ISONONANOATE | De 1 à 10 % |
| STEARATE D'ISOCETYLE | De 1 à 10 % |
| PEG 40 STEARATE | De 1 à 5 % |
| PEG 5 GLYCERYL STEAR | De 1 à 5 % |
| CONSERVATEURS | De 0 à 1 % |
| ALCOOL CETYLIQUE C16 C18 | De 0 à 2 % |
| PPG/SMDI POLYMERE | De 0 à 1 % |
| ACIDE SALICYLIQUE | De 0 à 2 % |
| SQUALANE GEL | De 0 à 2 % |
| DIOCTYL ETHER | De 1 à 10 % |
| ALCOOL DI-MALATE | De 1 à 10 % |
| EXTRAIT DE TOURNESOL | De 1 à 10 % |
| TROMETHAMINE | De 1 à 5 % |
| BUTYLENE GLYCOL | De 1 à 10 % |
| CITRATE TRISODIQUE | De 0 à 1 % |
| SCLEROTIUM GUM | De 0 à 1 % |
| AMIDON DE RIZ | De 1 à 10 % |
| POLYACRYLAMIDE GEL | De 0 à 1 % |
| VITAMINE C | De 0 à 2 % |
| GLYCINE | De 0 à 2 % |
| PARFUM | De 0 à 1 % |
| VITAMINE E | De 0 à 2 % |
| ACIDE CITRIQUE | De 0 à 1 % |
| SEPIWHITE | De 0 à 2 % |
| *CONCENTRAT DE PASSIFLORE* | De 0,01 à 20 % |
| EAU PURIFIÉE | QSP 100 % |

**STICK ROLL-ON ANTI-BACTERIEN:**

| **Matière première / Nom commercial ou Nom INCI** | **%** |
|---|---|
| EAU PURIFIÉE | QSP 100 % |
| BUTYLENE GLYCOL | De 1 à 5 % |
| PEROXYDE DE BENZOYLE | De 0 à 2 % |
| CAPRILOYL GLYCINE | De 0 à 5 % |
| ZINC PCA | De 0 à 5 % |
| *CONCENTRAT DE PASSIFLORE* | De 0,1 à 20 % |
| CARBOMERE | De 0 à 2 % |
| CONSERVATEURS | De 0 à 1 % |
| ACIDE CITRIQUE | De 0 à 1 % |
| TROMETHAMINE | De 0 à 1 % |

**SOIN GOMMANT :**

| **Matière première / Nom commercial ou Nom INCI** | **%** |
|---|---|
| ARLATONE DUO | De 5 à 20 % |
| AGENT GOMMANT | De 1 à 10 % |
| SCLEROTIUM GUM | De 1 à 10 % |
| CONSERVATEURS | De 0 à 1 % |
| CAPRYLOYL GLYCINE | De 0 à 1 % |
| SOUDE | De 0 à 1 % |
| *CONCENTRAT DE PASSIFLORE* | De 0,01 à 20 % |
| SEQUESTRANT | De 0 à 1 % |
| ACIDE CITRIQUE | De 0 à 1 % |
| EAU PURIFIÉE | QSP 100 % |
| PARFUM | De 0 à 1 % |

**FLUIDE KERATINISANT :**

| **Matière première / Nom commercial ou Nom INCI** | **%** |
|---|---|
| CETYL ALCOHOL | De 1 à 5 % |
| SILICONE 345 | De 1 à 5 % |
| ANTI-OXYDANT | De 0 à 1 % |
| EAU PURIFIEE | QSP 100 % |
| CETRIMONIUM CHLORIDE | De 0 à 5 % |
| QUINIE | De 0 à 5 % |
| VITAMINE B5 | De 0 à 5 % |
| *CONCENTRAT DE PASSIFLORE* | De 0,01 à 20 % |
| HYDROLYZED WHEAT PROTEIN | De 0 à 1 % |
| CONSERVATEUR | De 0 à 2 % |
| PARFUM | De 0 à 1 % |
| AJUSTEUR pH | De 0 à 1 % |

**SHAMPOOING ANTIPELLICULAIRE :**

| **Matière première / Nom commercial ou Nom INCI** | **%** |
|---|---|
| EAU PURIFIÉE | QSP 100 % |
| LAUROAMPHOACETATE | De 5 à 20 % |
| COCOGLUCOSIDE | De 5 à 20 % |
| DISTEARATE DE PEG 6000 | De 1 à 5 % |
| CONSERVATEURS | De 0 à 2 % |
| VITAMINE F | De 0 à 5 % |
| PIROCTONE OLAMINE | De 0 à 2 % |
| *CONCENTRAT DE PASSIFLORE* | De 0,01 à 20 % |
| ZINC PYRITHIONE | De 0 à 1 % |
| AJUSTEUR pH | De 0 à 1 % |
| SEQUESTRANT | De 0 à 1 % |
| PARFUM | De 0 à 1 % |

**FLUIDE DEMELANT :**

| **Matière première / Nom commercial ou Nom INCI** | **%** |
|---|---|
| CETEARYL ALCOHOL CETEARETH - 33 | De 1 à 5 % |
| QUATERNIUM-82 | De 0 à 2 % |
| EAU PURIFIEE | QSP 100 % |
| HYDROLYZED WHEAT PROTEIN | De 0 à 5% |
| CONSERVATEURS | De 0 à 2 % |
| AJUSTEUR pH | De 0 à 1 % |
| PARFUM | De 0 à 1 % |
| CYSTEINE | De 0 à 5 % |
| *CONCENTRAT DE PASSIFLORE* | De 0,01 à 20 % |

**LOTION CAPILLAIRE FORTIFIANTE :**

| **Matière première / Nom commercial ou Nom INCI** | **%** |
|---|---|
| EAU PURIFIEE | QSP 100 % |
| METHYL PROPANEDIOL | De 5 à 20% |
| CONSERVATEUR | De 0 à 2 % |
| AJUSTEUR pH | De 0 à 1 % |
| PARFUM | De 0 à 1 % |
| BIOTINE | De 0 à 5 % |
| VITAMINE B9 | De 0 à 5 % |
| *CONCENTRAT DE PASSIFLORE* | De 0,01 à 20 % |
| BETA-SITOSTEROL | De 0 à 1 % |
| ETHYLHEXYL COCOATE | De 0 à 5% |
| PEG-40 CASTOR OIL | De 0 à 5% |

**STICK PHOTOPROTECTEUR :**

| **Matière première / Nom commercial ou Nom INCI** | **%** |
|---|---|
| HUILE DE RICIN | QSP 100% |
| ALCOOL OLEIQUE | De 10 à 20% |
| HUILE PALMISTE | De 10 à 20% |
| POLYGLYCERIN-3-BEEWAX | De 10 à 20% |
| CIRE DE CANDELILLA | De 10 à 20% |
| HECTORITE | De 10 à 20% |
| TITANIUM DIOXYDE | De 0 à 5% |
| *CONCENTRAT DE PASSIFLORE* | De 0,01 à 20 % |
| BEURRE DE KARITE | De 0 à 5% |
| VITAMINE E | De 0 à 1% |

**CREME SOLAIRE SPF 50+ :**

| **Matière première / Nom commercial ou Nom INCI** | **%** |
|---|---|
| EAU PURIFIÉE B4 | QSP 100% |
| OXYDE DE TITANE | De 10 à 20% |
| CYCLOPENTASILOXANE | De 5 à 15% |
| OCTYL PALMITATE | De 5 à 15% |
| C12-C15 ALKYL BENZOATE | De 5 à 10% |
| DECYL PENTANOATE | De 5 à 10% |
| OXYDE DE ZINC | De 5 à 10% |
| GLYCEROL | De 1 à 5% |
| PEG-45/DODECYL GLYCOL COPOLYMERE | De 1 à 5% |
| *CONCENTRAT DE PASSIFLORE* | De 0,01 à 20 % |
| CHLORURE DE SODIUM | De 1 à 5% |
| DEXTRIN PALMITATE | De 1 à 2% |
| VITAMINE E | De 0 à 2% |
| CONSERVATEURS | De 0 à 2% |
| HYDROXYPROPYL GUAR | De 0 à 1 % |
| ALOE VERA | De 0 à 1% |
| LESSIVE SOUDE | De 0 à 1 % |
| EDTA 2 Na | De 0 à 1% |
| GLUCONATE ZINC | De 0 à 1% |

**SPRAY SOLAIRE SPF 50+ :**

| **Matière première / Nom commercial ou Nom INCI** | **%** |
|---|---|
| CAPRYOYL CAPRATE DE GLYCEROL | De 5 à 20% |
| CYCLOPENTASILOXANE | De 10 à 20% |
| DICAPRYLYL CARBONATE | De 5 à 20% |
| TINOSORB S | De 1 à 10% |
| OXYDE DE TITANE 100 | De 10 à 20% |
| HECTORITE | De 0 à 5% |
| ALPHA TOCOPHEROL | De 0 à 2% |
| LAURYLGLUCOSIDE-GLYSTEARATE | De 0 à 10% |
| EAU PURIFIÉE B4 | QSP 100% |
| ACIDE CITRIQUE | De 0 à 2% |
| PENTYLENE GLYCOL | De 0 à 5% |
| GLYCEROL | De 0 à 5% |
| GOMME XANTHANE | De 0 à 2% |
| *CONCENTRAT DE PASSIFLORE* | De 0,01 à 20 % |
| ALOE VERA | De 0 à 1% |
| GLUCONATE ZINC | De 0 à 1% |
| CONSERVATEURS | De 0 à 2% |
| TINOSORB M | De 1 à 10% |

**VERNIS POUR ONGLES FRAGILES ET CASSANTS :**

| **Matière première / Nom commercial ou Nom INCI** | **%** |
|---|---|
| ACRYLATE COPOLYMER | De 15 à 30 % |
| ETHANOL | QSP 100% |
| ACETONE | De 5 à 20 % |
| *CONCENTRAT DE PASSIFLORE* | De 0,01 à 5 % |

### Exemple 3 : Tests d'activités biologiques de l'extrait selon l'invention

L'extrait testé est le concentrât de l'exemple 1. Il sera nommé dans les études « concentrât de passiflore ». Sauf indications contraires, les pourcentages sont exprimés en poids de concentrât par rapport au poids total de la composition testée.

### A. Activité in vitro - modulation d'expression de gènes

L'analyse de la modulation d'expression de gènes de l'huile concentrée en fraction insaponifiable - appelé ensuite concentrât de passiflore, a été réalisée dans deux modèles différents : des fibroblastes humains normaux et des épidermes humains reconstruits mélanisés.

### 1) Effet sur l'expression de gènes dans des fibroblastes humains normaux

Les effets du concentrât de Passiflore ont été mesurés dans des fibroblastes humains normaux sur l'expression de gènes impliqués dans la biologie du derme, le remodelage des tissus conjonctifs et le vieillissement par qRT-PCR sur cartes TaqMan microfluidiques.

### a. Matériel et Méthodes

Des fibroblastes dermiques humains normaux ont été incubés pendant 24 heures en présence du concentrât de Passiflore à 0,01% et 0,05%. A la fin de l'incubation, les cellules ont été lysées et les ARNs totaux extraits puis dosés. Les différences d'expression géniques ont été analysées par RT-PCR quantitative à l'aide de cartes TaqMan. L'analyse des modifications d'expression et les analyses statistiques ont été réalisées à l'aide du logiciel Data Assist (Applied Biosystems).

### b. Résultats

Le concentrât de Passiflore a significativement modulé l'expression des gènes impliqués dans les fonctions suivantes (cf tableau 7) :

**Tableau 7 : Liste des gènes dont l'expression varie de manière significative en présence de concentrât de Passiflore dans des fibroblastes humains normaux**

| RQ = quantité relative d'expression du gène par rapport au témoin non traité (RQ=1) | | | | |
|---|---|---|---|---|
| | Concentrat 0,01% | | Concentrat 0,05% | |
| Nom des gènes | RQ | valeur de p | RQ | valeur de p |
| synthase 2 de l'acide hyaluronique | 2,6872 | 0,0276 | - | - |
| Fibrilline-1 | 2,3906 | 0,0047 | - | - |
| procollagène-lysine 2-oxoglutarate 5-dioxygenase 1 (Lysyl hydroxylase (LH1)) | 2,1811 | 0,0253 | - | - |
| NAD(H)dehydrogénase, quinone 1 | 2,1395 | 0,0405 | 2,1312 | 0,0153 |
| Fibromoduline | 2,078 | 0,0339 | - | - |
| Sous-unité alpha 1 du collagène 1 subunit (COL1A1) | 1,6491 | 0,0248 | 1,4726 | 0,0091 |
| Catalase | - | - | 1,9473 | 0,0216 |
| Sous-unité alpha 1 du collagène 7 (COL7A1) | - | - | 1,6802 | 0,0137 |
| Sous-unité alpha 1 du collagène 4 (COL4A1) | - | - | 1,5343 | 0,0359 |
| Mitochondrial peptide methionine sulfoxide reductase | - | - | 1,5218 | 0,0375 |
| Lumican | - | - | 1,4417 | 0,0441 |
| Glutathione synthétase | - | - | 1,4294 | 0,016 |
| Fibronectine | - | - | 1,401 | 0,0419 |
| Matrix metalloproteinase 3 (stromelysin 1) | - | - | 1,328 | 0,043 |

### 1) Réponse anti-oxydante

Le concentrât de Passiflore a induit l'expression des enzymes anti-oxydantes Mitochondrial peptide methionine sulfoxide reductase, NADH dehydrogénase, catalase (dont l'activité diminue au cours du vieillissement) et glutathion synthétase qui catalyse la synthèse du glutathion (molécule anti-oxydante). Ainsi par l'augmentation de ces gènes, le concentrât de Passiflore contribue à protéger le derme des dommages oxydatifs.

### 2) Homéostasie de la matrice extra-cellulaire

Le concentrât de Passiflore a induit l'expression de la hyaluronane synthase 2, enzyme impliquée dans la synthèse d'acide hyaluronique, polysaccharide connu pour sa capacité à retenir l'eau, à supporter l'architecture des tissus et l'élasticité de la peau et pour réguler la migration cellulaire. Ainsi le concentrât de Passiflore augmente la biosynthèse d'acide hyaluronique et donc favorise l'hydratation du derme et son élasticité.

Le concentrât de Passiflore a induit l'expression des gènes codant pour les collagènes de type 1, 4 et 7, la fibrilline (composant des fibres élastiques), la lysyl hydroxylase 1 (enzyme impliquée dans la formation des fibres élastiques), la fibromoduline et le lumican (petits protéoglycans qui régulent la formation de fibrilles), la fibronectine et la MMP3 (impliquée dans le remodelage du réseau de collagène). Ainsi le concentrât de Passiflore contribue à augmenter la fibrillogénèse et le remodelage de la matrice extracellulaire du derme pour permettre une meilleure cohésion et élasticité du derme.

### 2) Effet sur l'expression de gènes dans des épidermes humains reconstruits mélanisés

Les effets du concentrât de Passiflore ont été évalués dans des épidermes humains reconstruits mélanisés par qRT-PCR sur cartes TaqMan microfluidiques sur l'expression de gènes impliqués dans la pigmentation.

### a. Matériel et Méthodes

Des épidermes reconstitués contenant des mélanocytes humains primaires (provenant d'un donneur de phototype foncé) ont été incubés pendant 24 heures en présence du concentrât de Passiflore à 0,01% et 0,05%. A la fin de l'incubation, les tissus ont été lysés et les ARNs totaux extraits puis dosés, les différences d'expression géniques ont été analysées par RT-PCR quantitative à l'aide de cartes TaqMan. L'analyse des modifications d'expression et les analyses statistiques ont été réalisées à l'aide du logiciel Data Assist (Applied Biosystems).

### b. Résultats

Le concentrât de Passiflore a significativement réprimé l'expression des gènes tyrosinase et MITF (Microphtalmia-associated transcription factor), tous deux impliqués dans la mélanogénèse (cf tableau 8). La tyrosinase est une enzyme clé de la production de mélanine par les mélanocytes tandis que MITF régule la différenciation des mélanocytes et la transcription d'enzymes impliquées dans le processus de mélanogénèse. Ces diminutions d'expression suggèrent une activité dépigmentante du concentrât de Passiflore. D'autre part, le concentrât de Passiflore a diminué l'expression du NGF, récepteur du nerve growth factor, une neurotrophine impliquée notamment dans l'inflammation neurogène.

**Tableau 8 : Liste des gènes dont l'expression varie de manière significative en présence de concentrât de Passiflore dans des épidermes humains reconstruits mélanisés**

| RQ = quantité relative d'expression du gène par rapport au témoin non traité (RQ=1) | | | | |
|---|---|---|---|---|
| | Concentrât de passiflore 0,01% | | Concentrât de passiflore 0,04% | |
| Nom des gènes | RQ | Valeur de p | RQ | Valeur de p |
| Petite protéine riche en proline 2A (small prolin-rich protein 2A) | - | - | 0,6922 | 0,0268 |
| Facteur de croissance du nerf beta | - | - | 0,4978 | 0,0231 |
| Tyrosinase | 0,8088 | 0,0417 | - | - |
| Facteur de transcription associé à la microphtalmie | 0,5867 | 0,0186 | - | - |

### B. Activité sur la matrice extra-cellulaire dermique

Le screening sur fibroblastes ayant montré un potentiel effet du concentrât de Passiflore sur l'homéostasie de la matrice dermique, nous avons cherché à confirmer ce potentiel en étudiant d'une part l'effet du **concentrât de Passiflore** sur la prolifération de fibroblastes et sur leur capacité à produire les composants majoritaires de la matrice extracellulaire. D'autre part, un modèle de fibroblastes de vergeture a été utilisé pour évaluer l'effet relaxant du concentrât de Passiflore sur les forces développées par ces cellules particulières.

### 1. Effet sur la prolifération de fibroblastes humains normaux

### a. Matériel et Méthodes

Des fibroblastes dermiques humains normaux ont été incubés pendant 72 heures dans du milieu de culture à 1% de SVF en présence de concentrât de Passiflore à 0,005% et 0,01% ou dans du milieu de culture à 10% de SVF (contrôle positif). A la fin de l'incubation, la prolifération cellulaire a été évaluée par dosage du BrdU par une méthode ELISA en chimiluminescence. Le BrdU (5-bromo-2'-deoxyuridine) est un analogue de la thymidine qui s'incorpore, au cours du cycle cellulaire, au sein de l'ADN des cellules proliférantes.

### b. Résultats

Le concentrât de Passiflore a significativement augmenté la prolifération de fibroblastes humains normaux (tableau 9).

**Tableau 9 : Prolifération de fibroblastes humains normaux, intensité de luminescence proportionnelle à la quantité de BrdU incorporée exprimée en pourcentage d'augmentation par rapport au contrôle non traité.**

| | | Contrôle (1% SVF) | Contrôle positif (10% SVF) | concentrât de Passiflore **0,005%** | concentrât de Passiflore **0,01%** |
|---|---|---|---|---|---|
| **Prolifération cellulaire** | Intensité de luminescence moyenne | 1506398 | 23575410 | 2741224 | 2615640 |
| | Augmentation (%) | | +1465% *** | +**82%** ** | **+74%** ** |

| | | | | | |
|---|---|---|---|---|---|
| **p<0,01 ; ***p<0,001 - Analyse de variance à un facteur suivie d'un test de Dunnett | | | | | |

### 2. Effet sur l'expression des composants de la matrice dermique

### a. Matériel et Méthodes

Des fibroblastes dermiques humains normaux ont été incubés pendant 24 heures dans du milieu de culture à 1% de SVF en présence de concentrât de Passiflore à 0,005% et 0,05% ou de TGFβ1 à 5 ng/ml (référence). A la fin de l'incubation, les ARNs totaux ont été extraits, dosés puis rétro-transcrits. L'expression génique du collagène I et de l'élastine a été analysée par RT-PCR quantitative en temps réel.

### b. Résultats

Le concentrât de Passiflore a significativement augmenté l'expression génique du collagène de type I et de l'élastine (tableau 10), macromolécules constitutives et majoritaires de la matrice extra-cellulaire dermique.

**Tableau 10 : Expression génique (en quantité relative) du collagène I et de l'élastine dans des fibroblastes humains normaux traités par le concentrât de Passiflore; pourcentage d'augmentation par rapport au contrôle non traité.**

| | Collagène I (Quantité Relative) | | Elastine (Quantité Relative) | |
|---|---|---|---|---|
| Contrôle | 1,00 | | 1,00 | |
| Référence (TGFβ1) | 1,30 | +30% ns | 1,40 | +40% * |
| Concentrât de Passiflore 0,005% | 1,44 | **+44%** ** | 1,58 | **+58%** ** |
| Concentrât de Passiflore 0,05% | 1,87 | **+87%** *** | 1,92 | **+92%** *** |

| | | | | |
|---|---|---|---|---|
| *p<0,05 ; **p<0,01 ; ***p<0,001 - Analyse de variance à un facteur suivie d'un test de Dunnett | | | | |

### 3. Effet relaxant dans un modèle de fibroblastes de vergetures

Le système GlaSbox® a été utilisé pour évaluer l'effet relaxant du concentrât de Passiflore sur des fibroblastes issus d'une vergeture récente (rouge).

Le système GlaSbox® permet de mesurer, au sein d'un derme équivalent, les forces contractiles développées par les fibroblastes.

Les fibroblastes issus de vergeture rouge développent des forces contractiles supérieures aux forces développées par des fibroblastes de peau saine.

### a. Matériel et Méthodes

Des fibroblastes de peau saine et des fibroblastes de vergeture rouge ont été mis en culture à partir de biopsies réalisées chez une même patiente. Les dermes équivalents ont été préparés en mélangeant les fibroblastes (de peau saine ou de vergeture rouge) à une solution de collagène. Ce mélange a été coulé dans les cuves rectangulaires de la GlasBox®. Un milieu contenant ou non le concentrât de Passiflore à 0,005% ou 0,05% a été ajouté. Dans chacune de ces cuves plongent deux lames flexibles en silicium. Le derme équivalent se développe ainsi entre deux lames équipées d'un système de jauge de contrainte (« capteur »). Sous l'influence de la force de rétraction développée par les fibroblastes, les lames de silicium se déforment ; cela se traduit par une variation de la valeur de la résistance électrique de la jauge de contrainte. Cette variation, mesurée en temps réel, est représentative de la force développée au sein du derme équivalent. Les forces isométriques ont ainsi été mesurées pendant 24 heures.

### b. Résultats

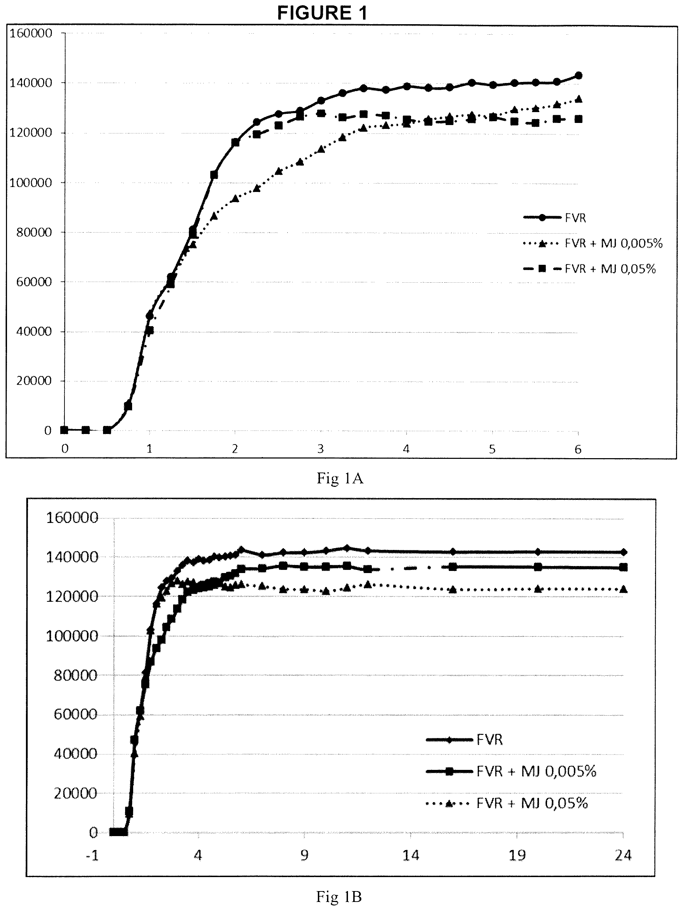

Les fibroblastes de vergeture rouge ont développé significativement plus de forces contractiles que les fibroblastes de la peau saine avoisinante (*résultats non montrés*). Le concentrât de Passiflore a significativement diminué les forces contractiles générées par les fibroblastes de vergeture rouge (figures 1A et 1B).

Figure 1 : Forces contractiles développées par des fibroblastes de vergeture rouge (FVR) en présence, ou non, de concentrât de Passiflore (MJ) au sein d'un derme équivalent sous tension dans le système GlaSbox®
A : détail des 6 premières heures ; B : courbes totales
   Statistiques : Analyse de variance à deux facteurs suivie d'un test de Fisher.
   ° FVR ; ∇ FVR + MJ 0,005% ; □ FVR + MJ 0,05%
   Axe des x : temps en heures ; axe des y : forces (unité arbitraire/million de cellule)

L'analyse des courbes Glasbox® (tableau 11) montre que l'AUC (aire sous la courbe), le maximum de contraction et la pente sont significativement diminués en présence du concentrât de Passiflore, celui-ci diminue donc non seulement les forces de contractions mais aussi la vitesse de contraction des fibroblastes de vergeture rouge.

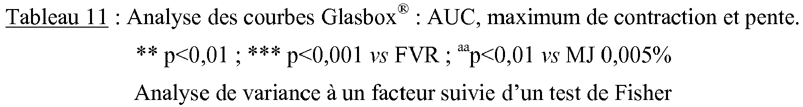

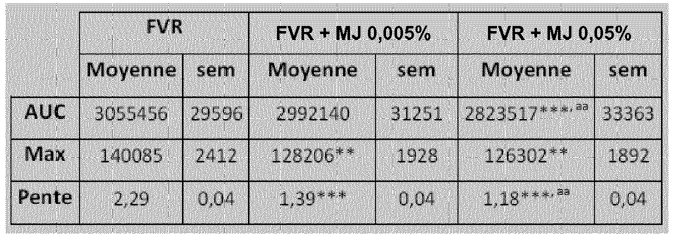

### 4. Conclusion

Ces résultats montrent l'effet anti-vergeture du concentrât de Passiflore par son action de diminution des forces contractiles générées par des fibroblastes de vergeture rouge ainsi que son effet redensifiant de la matrice dermique.

### C. Effet amincissant : effet sur la lipolyse du tissu adipeux humain

Le tissu adipeux blanc humain exerce une fonction métabolique fondamentale en fournissant aux autres tissus de l'organisme des molécules énergétiques sous forme d'acides gras libérés par le processus de lipolyse adipocytaire : l'adipocyte mobilise ses réserves énergétiques en hydrolysant les triglycérides stockés en acides gras et glycérol ; les acides gras ainsi libérés dans le sang sont utilisables par les autres tissus à des fins énergétiques. L'effet du concentrât de Passiflore sur la lipolyse adipocytaire a été évalué par le dosage de glycérol libéré lors de l'hydrolyse des triglycérides stockés dans des adipocytes matures humains cultivés en 3 dimensions.

### a. Matériel et Méthodes

Des adipocytes matures, issus de biopsies de tissu adipeux sous-cutané provenant de cinq donneurs (normo-pondérés ou en surpoids), ont été isolés et encapsulés dans un gel peptidique permettant le maintien de leur survie et de leur fonctionnalité biologique en culture. Les adipocytes matures encapsulés ont été incubés en présence du concentrât de Passiflore à 0,001% ou 0,005% ou de forskoline à 10 µM (référence) pendant 4 heures.

A la fin de l'incubation, le glycérol libéré a été dosé dans le milieu de culture au moyen d'un kit de dosage (Randox), les valeurs obtenues ont été normalisées par la quantité d'ADN évaluée à l'aide d'un kit (Invitrogen).

### b. Résultats

La libération de glycérol par les adipocytes a été augmentée par le concentrât de Passiflore chez chacun des 5 donneurs étudiés. Le tableau 6 présente la moyenne de l'effet observé sur les 5 donneurs : le concentrât de Passiflore a significativement stimulé la libération de glycérol, il favorise donc la lipolyse adipocytaire en faveur d'un effet amincissant.

**Tableau 12 : Effet lipolytique du concentrât de Passiflore évalué par la libération de glycérol par des adipocytes matures. Moyenne ± écart-type de 5 donneurs.**

| | Contrôle | Forskoline (référence) | Concentrât de Passiflore **0,001%** | Concentrât de Passiflore **0,005%** |
|---|---|---|---|---|
| Libération de glycérol (Unité artbitraire) | 1,00 ± 0,00 | 6,60 ± 1,54 | **1,40 ± 0,38** | **1,78 ± 0,30** |
| Stimulation (%) | | +660% ** | **+40% ns** | **+78%** ** |

| | | | | |
|---|---|---|---|---|
| **p<0,01 - Test non paramétrique de Wilcoxon/Kruskal Wallis | | | | |

## Revendications

1. Extrait lipidique de graines de Passiflores, *Passiflora incarnata* et/ou *Passiflora edulis,* préférentiellement de *P. edulis,* **caractérisé en ce que** ledit extrait lipidique est une huile de graines de Passiflores concentrée en sa fraction insaponifiable contenant de 3.0% à 100% en poids d'insaponifiables, par rapport au poids total de l'extrait.

2. Extrait selon la revendication 1, **caractérisé en ce que** l'extrait lipidique est une huile de graines de Passiflores concentrée en sa fraction insaponifiable présentant les caractéristiques suivantes ;
| Coupe grasse (% en poids par rapport au poids total de l'huile) | |
|---|---|
| C14 (acide myristique) | ≤ 1,0 |
| C16 (acide palmitique) | 5,0 -15,0 |
| C16' (acide 5-hexadécénoïque) | ≤ 1,0 |
| C18 (acide stéarique) | ≤ 5,0 |
| C18' (acide oléique) | 10,0 - 20,0 |
| C18" (acide linoléique) | 60,0 - 80,0 |
| C18''' (acide α-linolénique) | < 1,0 |
| C20 (acide arachidique) | ≤ 1,0 |
| C20' (acide eicasénoïque) | ≤ 1,0 |
| C22 (acide béhénique) | ≤ 1,0 |
| Teneur totale en Insaponifiables (g/100 g) | 3,0-15,0 |

3. Extrait selon la revendication 1, **caractérisé en ce que** l'extrait lipidique est un insaponifiable ayant les caractéristiques suivantes :
| Fraction insaponifiable (% massique par rapport au poids total de la fraction insaponifiable) | |
|---|---|
| Teneur en Tocophérols | 0,1 % - 3,0% |
| Teneur en Tocotriénols | 5,0 % - 25% |
| Teneur en Stérols | 30% - 60% |
| Teneur en Squalène | 10% - 35% |
| Non identifiés | < 30% |

4. Extrait selon l'une quelconque des revendications précédentes, **caractérisé en ce que** l'extrait lipidique est obtenu par un procédé comprenant les étapes successives suivantes :
a) distillation moléculaire d'une huile brute ou raffinée de passiflore ;
b) le cas échéant, extraction de l'insaponifiable, avantageusement par ;
i. saponification de l'huile de passiflore concentrée en sa fraction insaponifiable obtenue suite à l'étape a)
ii. puis extraction de l'insaponifiable à l'aide d'un solvant approprié,
c) récupération de l'huile concentrée en insaponifiable obtenus suite à l'étape a) ou de l'insaponifiable obtenu suite à l'étape b).

5. Procédé de préparation d'un extrait selon l'une quelconque des revendications précédentes, comprenant les étapes successives suivantes :
a) distillation moléculaire d'une huile brute ou raffinée de passiflore ;
b) le cas échéant, extraction de l'insaponifiable avantageusement par ;
i. saponification de l'huile de passiflore concentrée en sa fraction insaponifiable obtenue suite à l'étape a)
ii. puis extraction de l'insaponifiable à l'aide d'un solvant approprié,;
c) récupération de l'huile concentrée en insaponifiable obtenus suite à l'étape a) ou de l'insaponifiable obtenu suite à l'étape b).

6. Procédé selon la revendication 5, **caractérisé en ce que** l'étape a) de distillation moléculaire est réalisée en utilisant un dispositif choisi parmi les distillateurs moléculaires de type centrifuge et les dispositifs moléculaires de type à film raclé.

7. Composition comprenant, en tant qu'actif, un extrait lipidique de graines de Passiflores selon l'une quelconque des revendications 1 à 4 et un excipient approprié.

8. Composition selon la revendication 7, **caractérisée en ce qu'**elle comprend de 0,01% à 20% en poids dudit extrait lipidique, par rapport au poids total de la composition.

9. Composition selon la revendication 7 ou 8 ou extrait selon l'une quelconque des revendications 1 à 4, pour son utilisation en tant que, ou dans une, composition dermatologique, pharmaceutique ou en tant que, ou dans un, aliment fonctionnel.

10. Composition ou extrait selon la revendication 9, pour son utilisation pour stimuler, restaurer ou réguler le métabolisme des cellules de la peau et des muqueuses et/ou pour son utilisation dans la prévention et/ou le traitement des troubles liés au tissu dermique.

11. Composition ou extrait selon la revendication 10, pour son utilisation dans une ou plusieurs des indications suivantes :
- en tant qu'agent cicatrisant ;
- pour prévenir une altération de, et/ou maintenir l'homéostasie de la peau ou des muqueuses ;
- en tant qu'agent antioxydant ;
- en tant qu'agent anti-inflammatoire.

12. Composition ou extrait selon la revendication 10, pour son utilisation pour favoriser la cicatrisation.

13. Composition ou extrait selon l'une quelconque des revendications 10 à 12, pour son utilisation dans la prévention et/ou le traitement des pathologies ou conditions choisies dans le groupe constitué par les cicatrices superficielles, les cheilites,, la peau après piqûres, les abrasions de la peau, les boutons et/ou les croûtes cutanés.

14. Procédé de soin cosmétique de la peau et/ou des phanères et/ou des muqueuses, en particulier les peaux fragiles et sensibles et les lèvres fragiles, en vue d'améliorer leur état et/ou leur aspect, comprenant l'administration, par voie orale ou topique, d'un extrait selon l'une quelconque des revendications 1 à 4 ou d'une composition selon l'une quelconque des revendications 7 ou 8.

15. Méthode de traitement cosmétique des peaux sèches, avec sensation de tiraillement, ou pour remodeler la silhouette, limiter l'effet « peau d'orange » comprenant l'administration, par voie orale ou topique, d'un extrait selon l'une quelconque des revendications 1 à 4 ou d'une composition selon l'une quelconque des revendications 7 ou 8.

16. Procédé de soin cosmétique selon la revendication 14 en vue d'améliorer la fermeté, l'élasticité ou la tonicité de la peau, ou en vue de renforcer les propriétés mécaniques de la peau et des muqueuses, en particulier pour lutter contre la peau flétrie, molle, distendue, affaissée et/ou amincie, et/ou renforcer et/ou restaurer l'élasticité ou la fermeté de la peau.

17. Procédé de soin cosmétique selon la revendication 14 dans lequel l'extrait selon l'une quelconque des revendications 1 à 4 ou la composition selon l'une quelconque des revendications 7 ou 8 sont utilisés en tant qu'agent amincissant et/ou anti cellulite, ou en tant qu'agent dépigmentant.

18. Procédé de soin cosmétique de la peau et/ou des phanères et/ou des muqueuses en vue de prévenir ou retarder le vieillissement cutané prématuré, en particulier photo-induit, avantageusement en vue de prévenir, réduire et/ou traiter les rides, les ridules ou une altération du microrelief, ou en vue de prévenir ou traiter les vergetures de la peau comprenant l'administration, par voie orale ou topique, d'un extrait selon l'une quelconque des revendications 1 à 4 ou d'une composition selon l'une quelconque des revendications 7 ou 8.

19. Aliment fonctionnel comprenant un extrait lipidique tel que défini à l'une quelconque des revendications 1 à 4.

## Patentansprüche

1. Lipidextrakt aus Samen von Passionsblumen, *Passiflora incarnata* und/oder *Passiflora edulis*, vorzugsweise von *P. edulis,* **dadurch gekennzeichnet, dass** der Lipidextrakt ein konzentriertes Öl von Passionsblumensamen in seinem unverseifbaren Anteil ist, der 3,0 Gew.-% bis 100 Gew.-% unverseifbare Stoffe im Verhältnis zum Gesamtgewicht des Extrakts enthält.

2. Extrakt nach Anspruch 1, **dadurch gekennzeichnet, dass** der Lipidextrakt konzentriertes Öl von Passionsblumensamen in seinem unverseifbaren Anteil ist, die folgenden Eigenschaften aufweist:
| Fettschnitt (Gew.-% im Verhältnis zum Gesamtgewicht des Öls) | |
|---|---|
| C14 (Myristinsäure) | ≤ 1,0 |
| C16 (Palmitinsäure) | 5,0 - 15,0 |
| C16' (5-Hexadecensäure) | ≤ 1,0 |
| C18 (Stearinsäure) | ≤ 5,0 |
| C18' (Ölsäure) | 10,0 - 20,0 |
| C18" (Linolsäure) | 60,0 - 80,0 |
| C18"' (α-Linolensäure) | < 1,0 |
| C20 (Arachinsäure) | ≤ 1,0 |
| C20' (Eicosanolsäure) | ≤ 1,0 |
| C22 (Behensäure) | ≤ 1,0 |
| Gesamtgehalt an unverseifbaren Stoffen (g/100 g) | 3,0 - 15,0 |

3. Extrakt nach Anspruch 1, **dadurch gekennzeichnet, dass** der Lipidextrakt ein unverseifbarer Stoff mit den folgenden Eigenschaften ist:
| Unverseifbarer Anteil (Massen-% im Verhältnis zum Gesamtgewicht des unverseifbaren Anteils) | |
|---|---|
| Tocopherolgehalt | 0,1% - 3,0% |
| Tocotrienolgehalt | 5,0% - 25% |
| Sterolgehalt | 30% - 60% |
| Squalengehalt | 10% - 35% |
| Nicht identifiziert | < 30% |

4. Extrakt nach einem der vorstehenden Ansprüche, **dadurch gekennzeichnet, dass** der Lipidextrakt durch ein Verfahren erzielt wird, das die folgenden nacheinander Schritte umfasst:
a) Molekulardestillation eines rohen oder raffinierten Passionsblumenöls;
b) gegebenenfalls Extraktion des unverseifbaren Stoffes vorteilhaft durch;
i. Verseifung des konzentrierten Passionsblumenöls in seinem unverseifbaren Anteil, der nach Schritt a) erzielt wird,
ii. dann Extraktion des unverseifbaren Stoffes mit einem geeigneten Lösungsmittel,
c) Gewinnung des nach Schritt a) erzielten als unverseifbarer Stoff konzentrierten Öls oder des nach Schritt b) erzielten unverseifbaren Stoffes.

5. Verfahren zur Herstellung eines Extrakts nach einem der vorstehenden Ansprüche, das die folgenden nacheinander Schritte umfasst:
a) Molekulardestillation eines rohen oder raffinierten Passionsblumenöls;
b) gegebenenfalls Extraktion des unverseifbaren Stoffes vorteilhaft durch;
i. Verseifung des konzentrierten Passionsblumenöls in seinem unverseifbaren Anteil, der nach Schritt a) erzielt wird,
ii. dann Extraktion des unverseifbaren Stoffes mit einem geeigneten Lösungsmittel;
c) Gewinnung des nach Schritt a) erzielten als unverseifbarer Stoff konzentrierten Öls oder des nach Schritt b) erzielten unverseifbaren Stoffes.

6. Verfahren nach Anspruch 5, **dadurch gekennzeichnet, dass** Schritt a) der Molekulardestillation unter Verwendung einer Vorrichtung durchgeführt wird, die unter Zentrifugal-Molekularbrennern und Molekular-Schabefilmvorrichtungen ausgewählt ist.

7. Zusammensetzung umfassend als Wirkstoff einen Lipidextrakt aus Passionsblumensamen nach einem der vorstehenden Ansprüche 1 bis 4 und einen geeigneten Hilfsstoff.

8. Zusammensetzung nach Anspruch 7, **dadurch gekennzeichnet, dass** sie 0,01 Gew.-% bis 20 Gew.-% des Lipidextrakts im Verhältnis zum Gesamtgewicht der Zusammensetzung umfasst.

9. Zusammensetzung nach Anspruch 7 oder 8 oder Extrakt nach einem der Ansprüche 1 bis 4 zur Verwendung als oder in einer dermatologischen pharmazeutischen Zusammensetzung oder als oder in einem funktionellen Lebensmittel.

10. Zusammensetzung oder Extrakt nach Anspruch 9 zur Verwendung bei der Stimulierung, Wiederherstellung oder Regulierung des Stoffwechsels von Haut- und Schleimhautzellen und/oder zur Verwendung bei der Vorbeugung und/oder Behandlung von Hautgewebestörungen.

11. Zusammensetzung oder Extrakt nach Anspruch 10 zur Verwendung bei einer oder mehreren der folgenden Indikationen:
- als Wundheilmittel;
- zur Verhinderung einer Veränderung und/oder Aufrechterhaltung der Homöostase der Haut oder der Schleimhäute;
- als Antioxidationsmittel;
- als entzündungshemmendes Mittel.

12. Zusammensetzung oder Extrakt nach Anspruch 10 zur Verwendung bei der Förderung der Wundheilung.

13. Zusammensetzung oder Extrakt nach einem der Ansprüche 10 bis 12 zur Verwendung bei der Vorbeugung und/oder Behandlung von Krankheiten oder Zuständen, die in der Gruppe bestehend aus oberflächlichen Narben, Cheilitis, Haut nach Stichen, Hautabschürfungen, Pickeln und/oder Hautschorf ausgewählt sind.

14. Verfahren zur kosmetischen Pflege der Haut und/oder der Hautanhangsgebilde und/oder der Schleimhäute, insbesondere der anfälligen und empfindlichen Haut und spröden Lippen zur Verbesserung ihres Zustands und/oder Aussehens, umfassend die orale oder topische Verabreichung eines Extrakts nach einem der Ansprüche 1 bis 4 oder einer Zusammensetzung nach einem der Ansprüche 7 oder 8.

15. Verfahren zur kosmetischen Behandlung von trockener Haut mit spannendem Gefühl oder zur Modellierung der Silhouette, wobei der "Orangenhaut"-Effekt begrenzt wird, umfassend die orale oder topische Verabreichung eines Extrakts nach einem der Ansprüche 1 bis 4 oder einer Zusammensetzung nach einem der Ansprüche 7 oder 8.

16. Kosmetisches Pflegeverfahren nach Anspruch 14 zur Verbesserung der Festigkeit, Elastizität oder des Tonus der Haut oder zur Stärkung der mechanischen Eigenschaften der Haut und der Schleimhäute, insbesondere zur Bekämpfung von welker, schlaffer, gedehnter, hängender und/oder verdünnter Haut und/oder zur Stärkung und/oder Wiederherstellung der Elastizität oder Festigkeit der Haut.

17. Kosmetisches Pflegeverfahren nach Anspruch 14, wobei der Extrakt nach einem der Ansprüche 1 bis 4 oder die Zusammensetzung nach einem der Ansprüche 7 oder 8 als Schlankheitsmittel und/oder Mittel gegen Cellulite oder als Depigmentierungsmittel verwendet werden.

18. Verfahren zur kosmetischen Pflege der Haut und/oder der Hautanhangsgebilde und/oder der Schleimhäute, um insbesondere lichtbedingter vorzeitiger Hautalterung vorzubeugen oder zu verzögern, vorteilhaft, um Falten, Fältchen oder Veränderungen des Mikroreliefs zu verhindern, zu reduzieren und/oder zu behandeln, oder um Schwangerschaftsstreifen der Haut vorzubeugen oder zu behandeln, umfassend die orale oder topische Verabreichung eines Extrakts nach einem der Ansprüche 1 bis 4 oder einer Zusammensetzung nach einem der Ansprüche 7 oder 8.

19. Funktionelles Lebensmittel, umfassend einen Lipidextrakt, wie in einem der Ansprüche 1 bis 4 definiert.

## Claims

1. A lipid extract from Passiflora seeds, *Passiflora incarnata* and/or *Passiflora edulis,* preferably *P. edulis,* **characterized in that** said lipid extract is oil of Passiflora seeds concentrated in its unsaponifiable fraction containing from 3.0 weight % to 100 weight % of unsaponifiables relative to the total weight of the extract.

2. The extract according to claim 1, **characterized in that** the lipid extract is oil of Passiflora seeds concentrated in its unsaponifiable fraction having the following characteristics:
| Fatty fraction (weight % relative to total oil weight) | |
|---|---|
| C14 (myristic acid) | ≤ 1.0 |
| C16 (palmitic acid) | 5.0 - 15.0 |
| C16' (5-hexadecenoic acid) | ≤ 1.0 |
| C18 (stearic acid) | ≤ 5.0 |
| C18' (oleic acid) | 10.0 - 20.0 |
| C18" (linoleic acid) | 60.0 - 80.0 |
| C18'" (α-linolenic acid) | < 1.0 |
| C20 (arachidic acid) | ≤ 1.0 |
| C20' (eicasenoic acid) | ≤ 1.0 |
| C22 (behenic acid) | ≤ 1.0 |
| Total unsaponifiable content (g/100 g) | 3.0 - 15.0 |

3. The extract according to claim 1, **characterized in that** the lipid extract is an unsaponifiable having the following characteristics:
| Unsaponifiable fraction (weight % relative to total weight of the unsaponifiable fraction) | |
|---|---|
| Tocopherol content | 0.1% - 3.0% |
| Tocotrienol content | 5.0% - 25% |
| Sterol content | 30% - 60% |
| Squalene content | 10% - 35% |
| Non-identified | < 30% |

4. The extract according to any one of the preceding claims, **characterized in that** the lipid extract is obtained using a process comprising the following successive steps:
a) molecular distillation of crude or refined Passiflora oil;
b) optionally, extraction of the unsaponifiable, advantageously by;
i. saponification of the Passiflora oil concentrated in its unsaponifiable fraction obtained after step a);
ii. followed by extraction of the unsaponifiable using a suitable solvent.
c) recovery of the oil concentrated in unsaponifiable obtained after step a) or of the unsaponifiable obtained after step b).

5. A process to prepare an extract according to any one of the preceding claims, comprising the following successive steps:
a) molecular distillation of crude or refined Passiflora oil;
b) optionally, extraction of the unsaponifiable advantageously by;
i. saponification of the Passiflora oil concentrated in its unsaponifiable fraction obtained after step a);
ii. followed by extraction of the unsaponifiable using a suitable solvent;
c) recovery of the oil concentrated in unsaponifiable obtained after step a) or of the unsaponifiable obtained after step b).

6. The process according to claim 5, **characterized in that** the molecular distillation step a) is performed using a device selected from among molecular distillation units of centrifugal type and molecular devices of scraped film type.

7. A composition comprising as active ingredient a lipid extract of Passiflora seeds according to any one of claims 1 to 4 and a suitable excipient.

8. The composition according to claim 7, **characterized in that** it comprises from 0.01 weight % to 20 weight % of said lipid extract relative to the total weight of the composition.

9. The composition according to claim 7 or 8 or extract according to any one of claims 1 to 4 for use as or in a dermatological, pharmaceutical composition, or as or in a functional food.

10. The composition or extract according to claim 9, for use to stimulate, restore or regulate the metabolism of skin or mucosa cells and/or for use in the prevention and/or treatment of disorders related to dermal tissue.

11. The composition or extract according to claim 10, for use in one or more of the following indications:
- as healing agent;
- to prevent deterioration of and/or to maintain homeostasis of the skin or mucosae;
- as antioxidant agent;
- as anti-inflammatory agent;

12. The composition or extract according to claim 10, for use to promote healing.

13. The composition or extract according to any one of claims 10 to 12, for use in the prevention and/or treatment of pathologies or conditions selected from the group formed by surface scarring, cheilitis, skin after stings, skin abrasions, spots and/or skin scabs.

14. A cosmetic care method for the skin and/or skin appendages and/or mucosae, in particular for fragile and sensitive skin and fragile lips, with a view to improving the condition and/or appearance thereof, comprising the administration via oral or topical route, of an extract according to any one of claims 1 to 4 or of a composition according to any one of claims 7 or 8.

15. The cosmetic treatment method for dry skin, with feeling of tightness, or to remodel the silhouette, limit "orange peel" effect, comprising the administration via oral or topical route, of an extract according to any one of claims 1 to 4 or of a composition according to any one of claims 7 or 8.

16. The cosmetic care method according to claim 14, with a view to improving firmness, elasticity or tonicity of the skin, or with a view to reinforcing the mechanical properties of the skin and mucosae, in particular to combat withered, flabby, slackened, sagging and/or thinned skin, and/or to reinforce and/or restore skin elasticity or firmness.

17. The cosmetic care method according to claim 14, wherein the extract according to any one of claims 1 to 4 or the composition according to any one of claims 7 or 8 are used as slimming and/or anti-cellulite agent, or as depigmenting agent.

18. The cosmetic care method for the skin and/or skin appendages and/or mucosae, with a view to preventing or delaying premature skin ageing, in particular photo-induced, advantageously with a view to preventing, reducing and/or treating wrinkles, lines or microrelief deterioration, or with a view to preventing or treating skin stretch marks, comprising the administration via oral or topical route, of an extract according to any one of claims 1 to 4 or of a composition according to any one of claims 7 or 8.

19. A functional food comprising a lipid extract such as defined in any one of claims 1 to 4.
